Europäisches Patentamt

European Patent Office

Office européen des brevets

(11) Publication number: **0 087 864**
**B1**

## EUROPEAN PATENT SPECIFICATION

(45) Date of publication of patent specification: **15.10.86**

(21) Application number: **83300621.6**

(22) Date of filing: **08.02.83**

(51) Int. Cl.⁴: **A 61 K 45/06,** A 61 K 31/785, A 61 K 31/715, A 61 K 31/455, A 61 K 31/20, A 61 K 31/23 // (A61K31/785, 31:715, 31:455, 31:23, 31:20)

(54) **Pharmaceutical composition.**

(30) Priority: **01.03.82 GB 8205935**

(43) Date of publication of application:
**07.09.83 Bulletin 83/36**

(45) Publication of the grant of the patent:
**15.10.86 Bulletin 86/42**

(84) Designated Contracting States:
**AT BE CH DE FR GB IT LI LU NL SE**

(56) References cited:

ROTE LISTE, 1963, Editio Cantor,
Aulendorf/Württ., DE

CHEMICAL ABSTRACTS, vol. 96, no. 25, June
21, 1982, page 87, no. 210945v, Columbus,
Ohio, US

CHEMICAL ABSTRACTS, vol. 77, no. 25,
December 18, 1972, page 34, no. 160245b,
Columbus, Ohio, US, T.A. MIETTINEN: "Effect
of nicotinic acid on the fecal excretion of
neutral sterols and bile acids"

(73) Proprietor: **EFAMOL LIMITED**
**71/74 Mark Lane**
**London E.C.3 (GB)**

(72) Inventor: **Horrobin, David Frederick**
**P.O. Box 10**
**Nuns'Island Montreal H3E 1J8 (CA)**

(74) Representative: **Caro, William Egerton et al**
**J. MILLER & CO. Lincoln House 296-302 High**
**Holborn**
**London WC1V 7JH (GB)**

(56) References cited:
CHEMICAL ABSTRACTS, vol. 98, no. 13, March
28, 1983, page 68, no. 101199a, Columbus,
Ohio, US

CHEMICAL ABSTRACTS, vol. 77, no. 25,
December 18, 1972, page 398, no. 164488f,
Columbus, Ohio, US

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

*Field of the Invention*

The invention relates to compositions for and methods of reducing blood cholesterol levels, a desideratum in many disease states and in the maintenance of health.

*Background*

There are a number of agents which lower cholesterol levels in the blood by binding to bile salts in the gastro-intestinal tract and directly enhancing cholesterol excretion in the faeces. Cholestyramine, colestipol and dextrans such as polidexide are examples of substances effective in this way.

Further, Illingworth et al in the Lancet for 7th February 1981 pp. 296—7 report use of colestipol plus nicotinic acid (niacin) against an inherited high blood-cholesterol condition, with 'dramatic' effect. No mechanism is discussed, the article suggesting simply that therapy, in addition to taking binders, may best be directed towards reducing lipoprotein synthesis, and saying that niacin has been reported to do that. Said article uses the term bile acid sequestrant, equivalent to bile salt binders as referred to herein.

The present inventor has considered this report. He has noted first that niacin is one of the two forms of Vitamin B3, the other being niacinamide; by an unknown mechanism it acts systematically to lower cholesterol levels in blood without any substantial effect on cholesterol excretion.

The inventor believes that the effect of niacin is due to an effect it has in stimulating prostaglandin (PG) synthesis, specifically PGE1 synthesis, and that this is part of the mechanism that leads to reduced cholesterol synthesis and hence reduced levels in the blood. It is for example known that PGE1 stimulates the formation of cyclic AMP (adenosine monophosphate) and that cyclic AMP inhibits cholesterol synthesis. Further, niacin, in addition to its blood cholesterol lowering effect, causes flushing and tingling, effects that the inventor has noted are also among those of stimulating prostaglandin synthesis, particularly PGE1 synthesis. Niacinamide, in contrast, though generally equivalent in its bodily effects to niacin, does not show this stimulating effect on PG synthesis, nor does it cause flushing and tingling or show a blood cholesterol lowering effect. Linkage of these facts as instances of the unusual existence of differences in properties between niacin and niacinamide, is a reason for the inventor believing that PG levels and blood cholesterol levels are linked. Finally, the inventor has noted, that essential fatty acids, particularly γ-linolenic acid (GLA) and dihomo-γ-linolenic acid (DGLA), also act systematically to lower blood cholesterol levels, the mechanism again being unknown; these acids of course are the starting materials for 1-series PG synthesis as considered in some detail below.

There is, further, reference at Chem. Abs. 164488f (77 398 (1972) Koori et al.) to α-linolenyl, γ-linolenyl and arachidonyl nicotinates as hypocholesterolemic agents.

*The Invention*

The inventor believes he has drawn the above observations together for the first time, and that (without limitation) they provide a rationale for proposing compositions for and methods of reducing blood cholesterol wherein bile salt binding agents or niacin or both are used in conjunction with GLA and/or DGLA. Such combination is believed to attack the high blood cholesterol levels in two ways, by both enhancing excretion and reducing synthesis, and gives a greater effect than either agent alone.

*Dose Ranges*

Suitable amounts of active materials, other than GLA and DGLA given later herein are:

| | |
|---|---|
| Bile salt binders (colestipol, cholestyramine, dextrans such as polidexide) | 1—200 g preferably 10—30 g daily |
| Niacin | 10 mg—20 g preferably 2—6 g daily |

*Further Materials*

Further materials that may be used with essential fatty acids and bile salt binders are those of prior proposals of the inventor, all tending to enhance 1-series PG production or, more broadly expressed, to influence the 1-series/2-series PG balance in the body in favour of 1-series PG's. These proposals include use of zinc, β-lactam antibiotics and other materials listed and discussed in published European Patent Specification No. A 0 003 407; use of penicillamine, phenformin and levamisole, and colchicine, Vinca alkaloids and other materials listed and discussed in published European Patent Specification No. A 0 004 770; use of vitamin C, ethanol and opiate antagonists listed and discussed in published European Patent Specification No. A 0 019 423; and use of 4-amino and 8-amino quinolines, acridines, quinine and other materials including spironolactone listed and discussed in published European Patent Specification No. A 0 035 856. Reference may be made to these specifications for the full listings, discussion and dosages, which are applicable in the present context also.

*General Discussion*

The significance of use of essential fatty acids, where the intention is to affect prostaglandin metabolism and through it other bodily processes, is that when raised prostaglandin levels are desired it is for various reasons not practical to administer naturally-occurring prostaglandins such as PGE1 and PGE2 to patients, certainly for any long period. Consequently, considerable attention has focused on the use of prostaglandin precursors including linolenic acid, γ-linolenic acid (GLA) and dihomo-γ-linolenic acid (DGLA).

Conversion of these materials in the body is believed to be as shown in the following diagram:

cis-Linoleic Acid

(9,12-octadecadienoic acid)

↓

GLA

(6,9,12-octadecatrienoic acid)

↓

DGLA ester reserves (small) ⇌ DGLA 8,11,14 eicosatrienoic acid ⟶ 1 series endoperoxides ↘ 1 series PG's

↓

Large AA ester reserves ⇌ AA (Arachidonic acid i.e. 5,8,11,14-eicosatetraenoic acid)

↘

2 series endoperoxides

↓

2 series PG's

The broad outline of this pathway is well known, and it brings out clearly that a major function of essential fatty acids (EFAs) is to act as precursors for prostaglandins, 1-series PGs being formed from dihomo-γ-linolenic acid (DGLA) and 2-series PGs from arachidonic acid (AA). DGLA and AA are present in food in only small quantites, and the major EFA in food is linoleic acid which is first converted to γ-linolenic acid (GLA) and then to DGLA and AA, the latter step being irreversible. The conversion of linoleic acid to GLA is a limiting step, adequate in the young and healthy body but often inadequate in ageing or in many disease states.

DGLA is the key substance. GLA is almost completely and very rapidly converted in the body to DGLA and so for practical purposes the administration of DGLA and GLA amounts to the same thing. DGLA can be converted to a storage form, changed to arachidonic acid and thence to PGs of the 2-series, or converted to PGs of the 1-series.

A balance between 1-series and 2-series PGs is, the inventor believes, significant in terms of overall control of the conversion pathways given above. Such control is not understood in detail but without restriction to the theory it appears first that PGE2 is able to enhance the formation of 1-series PGs, and second that PGE1 is able to block arachidonic acid mobilisation from tissue stores. Thus the conditions for a

3

negative feedback control loop exist; overproduction of PGE2 from AA will activate PGE1 synthesis, the PGE1 will inhibit AA mobilisation, and production of 2-series PGs will drop. Further TXA2, an unstable product of the 2-series endoperoxides arising in 2-series production, also appears to limit AA mobilisation and to enhance 1-series PG and in particular PGE1 production. Thus again the activity of the 2-series PG synthesis pathway gives rise indirectly to a material that controls that pathway.

All of the above should be borne in mind when considering the application of the present invention.

*Packs*

If it is not desired to have compositions comprising the active materials together, as listed above, packs may be prepared comprising the materials presented for separate or part joint and part separate administration in the appropriate relative amounts, and such packs are within the purview of the invention.

*Dietary Compositions*

The invention is chiefly described in terms of pharmaceutical compositions, but it will be understood that the γ-linolenic and other acids, being in the nature of dietary supplements, could be incorporated in a dietary margarine or other foodstuffs; such foodstuffs, possibly containing other active materials and generally referred to in this description as dietary or pharmaceutical compositions, are within the purview of the invention and thus of the term pharmaceutical compositions, packs or the like used in the claims.

*Veterinary Applications*

It will be understood that where a disorder of a kind calling for treatment in animals arises, the invention while described primarily in terms of human medicine and treatment is equally applicable in the veterinary field.

*Amount of γ-Linolenic and Other Acids Specifically*

A preferred daily dosage for all purposes for an adult (weight ca 75 kg) is from 0.05 to 0.1 up to 1, 2, 5 or even 10 g as required of γ-linolenic acid or equivalent weight, calculated as γ-linolenic acid or a physiologically functional derivative thereof. Amounts in particular may be 0.1 to 1.0 g daily. Such doses correspond to about 2 to 20 g daily of the Oenothera oil discussed below. In place of, or in addition to, γ-linolenic acid, one may uuse dihomo-γ-linolenic acid or a physiologically functional derivative thereof, in amounts equivalent in molar terms to γ-linolenic acid and calculated as such. Other EFA's are likewise related back to γ-linolenic acid in molar terms. The dosage can for example be taken as a single dose or divided into 2, 3 or 4 subdivisions thereof as convenient.

Suitable physiologically functional derivatives, convertible in the body to GLA or DGLA to enter the biosynthetic pathway given earlier herein, are physiologically acceptable salts, esters (particularly glycerides and simple $C_1$—$C_4$ alkyl esters), amides and phospholipids. Indirect identification of useful derivatives is by their having the valuable effect in the body of the acid (GLA or DGLA) itself, but conversion can be shown directly by gas chromatographic analysis of GLA or DGLA concentration in blood, body fat, or other tissue by standard techniques for example those of Pelick et al. p. 23, "Analysis of Lipids and Lipoproteins" Ed. Perkins, American Oil Chemists Society, Champaign, Illinois, U.S.A. (1975).

If desired, pharmaceutical compositions may be produced for use in the invention by associating natural or synthetic γ-linolenic acid (or a physiologically functional derivative thereof) and/or dihomo-γ-linolenic acid (or a physiologically functional derivative thereof), as such, with an acceptable pharmaceutical vehicle. It is at present convenient to incorporate the γ-linolenic acid into compositions in the form of an available oil having a high γ-linolenic acid content, hence references to "oil" herein.

At the present time known natural sources of oils having a high γ-linolenic acid content are few (there are no known natural sources of significant amounts of dihomo-γ-linolenic acid). One source of oils currently available is the seed of Evening Primrose species such as *Oenothera biennis L.* and *Oenothera lamarckiana*, the oil extract therefrom containing γ-linolenic acid (about 8%) and linoleic acid (about 72%) in the form of their glycerides together with other glycerides (percentages based on total fatty acids). Another source of γ-linolenic acid is Borage species such as *Borago officinalis* which, though its current yield per acre is low, provides a richer source of γ-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglycerides of γ-linolenic acid and linoleic as the main fatty acid components, the γ-linolenic acid content being if desired a major proportion. Seed oil extracts appear to have a stabilising effect upon any dihomo-γ-linolenic acid or physiologically functional derivative thereof.

*Pharmaceutical Presentation*

The compositions according to the invention are conveniently in a form suitable for oral, rectal, parenteral or topical administration in a suitable pharmaceutical vehicle, as discussed in detail for example in U.K. Patent Specification No. 1,082,624, to which reference may be made, and in any case very well known generally for any particular kind of preparation. Thus for example tablets, capsules, ingestible liquid or powder preparations, creams or lotions for topical application, or suppositories, can be prepared as required. Injectable solutions of hydrolysed Oenothera oil may be prepared using albumin to solubilise the free acid.

Advantageously a preservative is incorporated into the preparations. α-Tocopherol in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions useful in treatment according to the invention.

### Examples

Pharmaceutical compositions contain a unit dose of an oil extract from the seeds of *Oenothera biennis L.* and one of the other active materials of the present invention, optionally with added DGLA for example as with methyl dihomo-γ-linolenate. They may be presented by encapsulation of the natural oil in soft gelatin capsules by known methods.

The oil is extracted from the seeds by one of the conventional methods of extraction such as cold pressure, screw pressure after partially cooking the seed, or solvent extraction.

Fractionation of a typical sample of this oil shows a yield of 97.0% oil in the form of methyl esters, with the relative proportions:

| | |
|---|---|
| Palmitate | 6.15 |
| Stearate | 1.6 |
| Oleate | 10.15 |
| Linoleate | 72.6 |
| γ-Linolenate | 8.9 |

As preservative, α-tocopherol is added to the oil in a concentration of 0.1%.

Gelatin capsules containing oil extracts prepared as described above, each having the following contents of active ingredients (0.5 g oil extract = ca 0.045 g γ-linolenic acid), are prepared in conventional fashion.

### Example 1

| | |
|---|---|
| Oil extract | 0.5 g |

Two capsules may be administered thrice daily in the treatment of high blood cholesterol levels, giving a daily dose of γ-linolenic acid of ca. 0.27 g, in conjunction with 10 g colestipol, cholestyramine or polidexide on each occasion.

### Example 2

| | |
|---|---|
| Oil extract | 0.5 g |
| Methyl dihomo-γ-linolenate | 10 mg |

Two capsules may be administered thrice daily in the treatment of high blood cholesterol levels, in conjunction with 5 g colestipol, cholestyramine or polidexide on each occasion.

### Example 3

| | |
|---|---|
| Oil extract | 0.5 g |
| Niacin | 1 g |

Two capsules may be administered thrice daily in the treatment of high blood cholesterol levels, alone or in conjunction with colestipol, cholestyramine or polidexide as in Examples 1 and 2.

**Claims for the Contracting States: BE CH DE FR GB IT LI LU NL SE**

1. A pharmaceutical composition comprising γ-linolenic acid or dihomo-γ-linolenic acid or

physiologically functional derivative thereof convertible in the body thereto in conjunction with a bile acid sequestrant or niacin or both, lithium salts being absent from the composition when the bile acid sequestrant is a dextran and is used without niacin.

2. A composition according to claim 1, wherein the bile acid sequestrant is colestipol, cholestyramine or polidexide or other dextran.

3. A composition according to claim 1 or 2 presented for administration to give 0.05 to 10 g γ-linolenic acid daily or molar equivalent amount of dihomo-γ-linolenic acid or derivative.

4. A composition according to any preceding claim presented for administration to give 1 to 200 g bile acid sequestrant daily.

5. A composition according to claim 4 wherein said amount is 10 to 30 g daily.

6. A composition according to any preceding claim presented for administration to give 10 mg to 20 g niacin daily.

7. A composition according to claim 6 wherein said amount is 2 to 6 g daily.

8. A pharmaceutical pack comprising the materials set out in any preceding claim presented separately, or one or more separately and others together, but for joint administration.

9. The composition of any of claims 1 to 7 or the pack of claim 8 for use to reduce blood cholesterol levels.

10. The use of γ-linolenic acid or dihomo-γ-linolenic acid or physiologically functional derivative thereof convertible in the body thereto in conjunction with a bile acid sequestrant or niacin or both for the method of providing a pharmaceutical composition for treatment to reduce blood cholesterol levels.

11. The use according to claim 10 wherein the bile acid sequestrant is colestipol, cholestyramine or polidexide or other dextran.

12. The use according to claim 10 or 11 wherein said composition is presented for administration to give 0.05 to 10 g γ-linolenic acid daily or molar equivalent amount of dihomo-γ-linolenic acid or derivative.

13. The use according to any preceding claim in which said composition is presented for administration to give 1 to 200 g bile acid sequestrant daily.

14. The use according to claim 13 wherein said amount is 10 to 30 g daily.

15. The use according to any preceding claim wherein said composition is presented for administration to give 10 mg to 20 g niacin daily.

16. The use according to claim 15 wherein said amount is 2 to 6 g daily.

**Claims for the Contracting State: AT**

1. The use of γ-linolenic acid or dihomo-γ-linolenic acid or physiologically functional derivative thereof convertible in the body thereto in conjunction with a bile acid sequestrant or niacin or both for the method of providing a pharmaceutical composition for treatment to reduce blood cholesterol levels.

2. The use according to claim 1 wherein the bile acid sequestrant is colestipol, cholestyramine or polidexide or other dextran.

3. The use according to claim 1 or 2 wherein said composition is presented for administration to give 0.05 to 10 g γ-linolenic acid daily or molar equivalent amount of dihomo-γ-linolenic acid or derivative.

4. The use according to any preceding claim in which said composition is presented for administration to give 1 to 200 g bile acid sequestrant daily.

5. The use according to claim 4 wherein said amount is 10 to 30 g daily.

6. The use according to any preceding claim wherein said composition is presented for administration to give 10 mg to 20 g niacin daily.

7. The use according to claim 6 wherein said amount is 2 to 6 g daily.

**Patentansprüche für die Vertragsstaaten: BE CH DE FR GB IT LI LU NL SE**

1. Pharmazeutische Zusammensetzung, bestehend aus γ-Linolensäure oder Dimono-γ-Linolensäure oder deren physiologisch wirksamen, im Körper dazu umwandelbarem Derivat in Verbindung mit einem Gallensäure-Komplexsalzbildner oder Niacin oder beidem, wobei keine Lithiumsalze in der Zusammensetzung vorliegen, wenn der Gallensäure-Komplexsalzbildner ein Dextran ist und ohne Niacin verwendet wird.

2. Zusammensetzung nach Anspruch 1, dadurch gekennzeichnet, daß es sich bei dem Gallensäure-Komplexsalzbildner um Colestipol, Cholestyramin oder Polidexid oder ein anderes Dextran handelt.

3. Zusammensetzung nach Anspruch 1 oder 2 in einer Form zur Verabreichung von 0,05 bis 10 g γ-Linolensäure oder der molar äquivalenten Mengen von Dihomo-γ-Linolensäure oder deren Derivat täglich.

4. Zusammensetzung nach einem der vorhergehenden Ansprüche in einer Form zur Verabreichung von 1 bis 200 g Gallensäure-Komplexsalzbildner täglich.

5. Zusammensetzung nach Anspruch 4, dadurch gekennzeichnet, daß die Tagesdosis 10 bis 30 g beträgt.

6. Zusammensetzung nach einem der vorhergehenden Ansprüche in einer Form zur Verabreichung von 10 mg bis 20 g Niacin täglich.

7. Zusammensetzung nach Anspruch 6, dadurch gekennzeichnet, daß die Tagesdosis 2 bis 6 g beträgt.

8. Pharmazeutische Abpackung mit den in einem der vorhergehenden Ansprüche angegebenen, getrennt abgebackten Substanzen oder mindestens einer getrennt abgepackten und anderen zusammengepackten Substanzen, jedoch zur gemeinsamen Verabreichung.

9. Zusammensetzung nach einem der Ansprüche 1 bis 7 oder Abpackung nach Anspruch 8 zur Anwendung für die Herabsetzung des Cholesteringehalts im Blut.

10. Anwendung von γ-Linolensäure oder Dihomo-γ-Linolensäure oder deren physiologisch wirksamen, im Körper dazu umwandelbarem Derivat in Verbindung mit einem Gallensäure-Komplexsalzbildner oder Niacin oder beidem für das Verfahren zur Herstellung einer pharmazeutischen Zusammensetzung für eine Behandlung zur Herabsetzung des Cholesteringehalts im Blut.

11. Anwendung nach Anspruch 10, dadurch gekennzeichnet, daß es sich bei dem Gallensäure-Komplexsalzbildner um Colestipol, Cholestyramin oder Polidexid oder ein anderes Dextran handelt.

12. Anwendung nach Anspruch 10 oder 11, dadurch gekennzeichnet, daß die Zusammensetzung in einer Form zur Verabreichung von 0,05 bis 10 g γ-Linolensäure oder der molar äquivalenten Menge von Dihomo-γ-Linolensäure oder deren Derivat dargeboten wird.

13. Anwendung nach einem der vorhergehenden Ansprüche, bei welcher die Zusammensetzung in einer Form zur Verabreichung von 1 bis 200 g Gallensäure-Komplexsalzbilner täglich dargeboten wird.

14. Anwendung nach Anspruch 4, dadurch gekennzeichnet, daß die Tagesdosis 10 bis 30 g beträgt.

15. Anwendung nach einem der vorhergehenden Ansprüche, dadurch gekennzeichnet, daß die Zusammensetzung in einer Form zur Verabreichung von 10 mg bis 20 g Niacin täglich dargeboten wird.

16. Anwendung nach Anspruch 6, dadurch gekennzeichnet, daß die Tagesdosis 2 bis 6 g beträgt.

**Patentansprüche für den Vertragsstaat: AT**

1. Verwendung von γ-Linolensäure oder Dihomo-γ-Linolensäure oder deren physiologisch wirksamen, im Körper dazu umwandelbarem Derivat in Verbindung mit einem Gallensäuresalz-Bindemittel oder Niacin oder beidem für das Verfahren zur Herstellung einer pharmazeutischen Zusammensetzung zur Herabsetzung des Cholesteringehalts im Blut.

2. Verwendung gemäß Anspruch 1, dadurch gekennzeichnet, daß als Gallensäuresalz-Bindemittel Colestipol, Cholestyramin oder Polidexid oder ein anderes Dextran vorgesehen ist.

3. Verwendung gemäß Anspruch 1 oder 2, dadurch gekennzeichnet, daß die Zusammensetzung in einer Form zur Verabreichung von 0,05 bis 10 g γ-Linolensäure täglich oder der molar äquivalenten Menge von Dihomo-γ-Linolensäure oder deren Derivat vorliegt.

4. Verwendung gemäß einem der vorhergehenden Ansprüche, dadurch gekennzeichnet, daß die Zusammensetzung in einer Form zur Verabreichung von 1 bis 200 g Gallensäuresalz-Bindemittel täglich vorliegt.

5. Verwendung nach Anspruch 4, dadurch gekennzeichnet, daß die Tagesdosis 10 bis 30 g beträgt.

6. Verwendung gemäß einem der vorhergehenden Ansprüche, dadurch gekennzeichnet, daß die Zusammensetzung in einer Form zur Verabreichung von 10 mg bis 20 g Niacin täglich vorliegt.

7. Verwendung gemäß Anspruch 6, dadurch gekennzeichnet, daß die Tagesdosis 2 bis 6 g beträgt.

**Revendications pour les Etats contractants: BE CH DE FR GB IT LI LU NL SE**

1. Une composition pharmaceutique comprenant de l'acide γ-linolénique ou de l'acide dihomo-γ-linolénique ou un dérivé fonctionnel physiologique de ces acides convertible en ces acides dans l'organisme, conjointement avec un séquestrant des acides biliaires ou de la niacine ou les deux, les sels de lithium étant absents de la composition lorsque le sequestrant des acides biliaires est un dextrane et qu'il est utilisé sans niacine.

2. Une composition selon la revendication 1, dans laquelle le séquestrant des acides biliaires est le colestipol, la cholestyramine ou le polydexide ou un autre dextrane.

3. Une composition selon la revendication 1 ou 2, présentée pour l'administration quotidienne de 0,05 à 10 g d'acide γ-linolénique ou de la quantité molaire équivalente d'acide dihomo-γ-linolénique ou d'un dérivé de cet acide.

4. Une composition selon l'une quelconque des revendications qui précèdent, présentée pour l'administration quotidienne de 1 à 200 g de séquestrant des acides biliaires.

5. Une composition selon la revendication 4, dans laquelle ladite quantité est de 10 à 30 g par jour.

6. Une composition selon l'une quelconque des revendications qui précèdent, présentée pour l'administration quotidienne de 10 mg à 20 g de niacine.

7. Une composition selon la revendication 6, dans laquelle ladite quantité est de 2 à 6 g par jour.

8. Un emballage pharmaceutique comprenant les matières énumérées dans l'une quelconque des revendications qui précèdent, présentées séparément ou bien une ou plusieurs séparément et les autres ensemble, mais pour l'administration conjointe.

9. La composition selon l'une quelconque des revendications 1 à 7, ou l'emballage de la revendication 8 pour l'utilisation en vue d'abaisser les taux de cholestérol sanguin.

10. L'utilisation de l'acide γ-linolénique ou de l'acide dihomo-γ-linolénique ou d'un dérivé fonctionnel physiologique de ces acides convertible en ces acides dans l'organisme, conjointement avec un séquestrant des acides biliaires ou la niacine ou les deux pour le procédé de préparation d'une composition pharmaceutique pour le traitement en vue d'abaisser les taux de cholestérol sanguin.

11. L'utilisation selon la revendication 10, dans laquelle le séquestrant des acides biliaires est le . colestipol, le cholestyramine ou le polydexide ou un autre dextrane.

12. L'utilisation selon la revendication 10 ou 11, dans laquelle la composition est présentée pour l'administration quotidienne de 0,05 à 10 g d'acide γ-linolénique ou de la quantité molaire équivalente d'acide dihomo-γ-linolénique ou d'un dérivé de cet acide.

13. L'utilisation selon l'une quelconque des revendications qui précèdent, dans laquelle la composition est présentée pour l'administration quotidienne de 1 à 200 g de séquestrant des acides biliaires.

14. L'utilisation selon la revendication 4, dans laquelle ladite quantité est de 10 à 30 g par jour.

15. L'utilisation selon l'une quelconque des revendications qui précèdent, dans laquelle ladite composition est présentée pour l'administration quotidienne de 10 mg à 20 g de niacine.

16. L'utilisation selon la revendication 6, dans laquelle ladite quantité est de 2 à 6 g par jour.

**Revendications pour l'Etat contractant: AT**

1. L'utilisation de l'acide γ-linolénique ou de l'acide dihomo-γ-linolénique ou d'un dérivé fonctionnel physiologique de ces acides convertible en ces acides dans l'organisme, conjointement avec un séquestrant des acides biliaires ou la niacine ou les deux pour le procédé de préparation d'une composition pharmaceutique pour le traitement en vue de diminuer les taux de cholestérol sanguin.

2. L'utilisation selon la revendication 1, dans laquelle le séquestrant des acides biliaires est le colestipol, le cholestyramine ou le polydexide ou un autre dextrane.

3. L'utilisation selon la revendication 1 ou 2, dans laquelle la composition est présentée pour l'administration quotidienne de 0,05 à 10 g d'acide γ-linolénique ou de la quantité molaire équivalente d'acide dihomo-γ-linolénique ou d'un dérivé de cet acide.

4. L'utilisation selon l'une quelconque des revendications qui précèdent, dans laquelle la composition est présentée pour l'administration quotidienne de 1 à 200 g de séquestrant des acides biliaires.

5. L'utilisation selon la revendication 4, dans laquelle ladite quantité est de 10 à 30 g par jour.

6. L'utilisation selon l'une quelconque des revendications qui précèdent dans laquelle ladite composition est présentée pour l'administration quotidienne de 10 mg à 20 g de niacine.

7. L'utilisation selon la revendication 6, dans laquelle ladite quantité est de 2 à 6 g par jour.